# DEMANDE DE BREVET EUROPEEN

(11) **EP 4 464 931 A1**
(43) Date de publication de la demande: **20.11.2024**
(21) Numéro de dépôt: 24176183.2
(22) Date de dépôt: 16.05.2024
(51) Int. Cl.: F21K 9/00, A61N 5/06

(54) **DISPOSITIF LUMINEUX À COMBINAISON DE LEDS**

(30) Priorité: 17.05.2023 FR 2304916
(71) Demandeur: Bye Blues, 67100 Strasbourg (FR)
(72) Inventeur: BOUDARD, Domitille, 67100 STRASBOURG (FR); VERRA, Daniela, 67200 STRASBOURG (FR)
(74) Mandataire: Cabinet Bleger-Rhein-Poupon

(57) **Abrégé**

L'invention concerne un dispositif lumineux à LEDs (1) pour la diffusion d'un signal lumineux, comprenant un logement incorporant au moins une première source lumineuse comportant au moins une LED émettant selon une première longueur d'ondes et une deuxième source lumineuse comportant au moins une LED, ledit logement étant conçu apte à permettre la diffusion du signal émis par lesdites sources lumineuses, ces dernières étant alimentées en énergie au moyen d'un boîtier d'alimentation électrique.

Ledit dispositif (1) est caractérisé en ce que la première longueur d'ondes émise par ladite première source lumineuse est égale à 670 nm et en ce que ladite deuxième longueur d'ondes émise par ladite deuxième source lumineuse est comprise entre 720 et 750 nm.

## Description

La présente invention concerne le domaine des dispositifs émettant un signal lumineux et, plus particulièrement, des dispositifs comprenant une combinaison d'au moins deux sources de lumière, chacune étant de type diode électroluminescente ou LED (« Light Emitting Diode ») émettant selon une longueur d'ondes spécifique.

L'objectif de la présente invention est plus particulièrement de proposer un dispositif lumineux destiné à percevoir son environnement et à s'éclairer pendant la phase nocturne, tout en évitant les effets néfastes potentiels de l'exposition aiguë ou chronique à la lumière de nuit sur l'apparition de troubles du sommeil, de troubles de l'humeur, de perturbations métaboliques, de pathologies cardio-vasculaires et de risques de cancer.

On connait dans l'état de la technique, notamment du document de brevet américain US 2012170264, des dispositifs lumineux de type lampe de culture à LEDs omnispectre, pour une optimisation de la croissance des plantes, dans les domaines de l'agriculture ou de l'horticulture.

De tels dispositifs, en particulier celui décrit dans le document susmentionné, sont composés d'une pluralité de lampes à LED, chacune présentant une longueur d'onde de sortie spécifique, en sorte de balayer l'intégralité du spectre lumineux dans le domaine du visible.

En d'autres termes, les LEDs de ce type de dispositif émettent à des longueurs d'ondes allant de plus ou moins 400 nm, à environ 800 nm, la lampe de culture étant, en effet, destinée à émettre une lumière multispectre aussi bien dans les tons bleus, que dans le vert, ou bien encore dans le rouge.

Ainsi, les lampes fabriquées de nos jours, et mises en oeuvre dans de nombreux domaines, utilisent en très grande majorité des LEDs comme composants émetteurs de lumière.

La plupart des lampes ayant pour objectif l'éclairage en vue d'améliorer la visibilité dans le cadre d'activités humaines, sont constantes dans leur type d'émission au cours des 24h de la journée et utilisent, dans la très grande majorité des cas, des LEDs émettant une lumière blanche (froide, neutre ou chaude).

Le spectre d'émission de ces LEDs couvre l'ensemble de la lumière visible avec généralement deux pics d'émission : l'un étroit dans le bleu à 450 nm et l'un plus large à environ 600 nm dans le jaune-orange.

La lumière blanche présente l'avantage de permettre la réalisation de tâches fines et complexes grâce à un très bon Indice de Rendu des Couleurs, ou IRC.

Cependant, cette lumière blanche présente l'inconvénient de ne pas prendre en compte l'impact photobiologique néfaste, pendant la nuit, d'une partie du spectre d'émission sur les systèmes cérébraux dont ceux générant et synchronisant les rythmes biologiques et les fonctions efférentes, à savoir, notamment, le sommeil, la cognition, et l'humeur, mais aussi sur des circuits ayant une influence directe (sans passer par le système circadien) sur l'humeur et l'éveil/la vigilance.

Ainsi, l'exposition nocturne à la lumière blanche a un impact néfaste sur plusieurs fonctions cérébrales et, par conséquent, sur le bien-être en général.

Une des voies cérébrales sous-jacente à cet effet est le contrôle de l'expression de la mélatonine dans la glande pinéale par les noyaux suprachiasmatiques participant à la régulation des rythmes biologiques chez l'humain.

De récents résultats d'expériences laissent aussi suggérer qu'il existe des voies de transduction du signal lumineux ayant des effets sur l'humeur et l'éveil, indépendantes du système circadien.

Certaines lampes sur le marché destinées à un usage nocturne sont commercialisées sur la base d'un argumentaire scientifique avec des références à la chronobiologie.

Les LEDs de ces lampes émettent généralement dans les zones orange et rouge de la partie visible du spectre.

Cela étant, malgré un argumentaire scientifique, certaines des lampes diffusant une lumière rouge-orangée émettent tout de même aussi dans une partie du spectre pouvant avoir une incidence sur certaines fonctions d'éveil.

D'autres lampes ont bien un spectre d'émission déclaré comme étant exclusif dans la partie du spectre "sûr" de nuit. Cependant, les spectres d'émission des LEDs étant étroits, ces lampes présentent souvent un inconvénient majeur : l'Indice de Rendu des Couleurs est très bas et donne un mauvais confort visuel compatible qui ne permet pas de bien voir de nuit.

C'est le cas notamment du dispositif lumineux qui est décrit dans la demande de brevet européen n° EP 3 519 030 et qui a pour fonction essentielle uniquement d'aider à l'endormissement et/ou le repos d'un utilisateur.

Ce dispositif émet une lumière monochromatique, ou quasi monochromatique, de couleur rouge, ayant une longueur d'onde préférentiellement de l'ordre de 625 nm, plus ou moins 15 nm, avec une puissance décroissante au cours du temps, depuis une puissance maximale jusqu'à une puissance minimale, en sorte que l'intensité lumineuse diminue continuellement et progressivement jusqu'à l'extinction.

La présente invention se veut à même de remédier, au moins en partie, aux inconvénients des dispositifs lumineux de l'état de la technique.

Plus particulièrement, le dispositif lumineux à présent mis au point permet d'obtenir un compromis satisfaisant de spectre d'émission qui soit, à la fois, adapté à la physiologie humaine nocturne (ayant le moins d'impact sur les structures cérébrales non-visuelles) tout en permettant un confort visuel compatible avec la réalisation de tâches, plus ou moins complexes, durant la nuit.

Le dispositif lumineux comporte alors, en tant que moyens d'éclairage, une combinaison d'au moins deux LEDs différentes dont la couverture des deux pics d'émission est assez large pour offrir un bon rendu visuel mais aussi située dans la zone "sûre" au niveau de son impact photobiologique.

L'utilisateur du dispositif peut alors effectuer des tâches basiques ou nécessitant une plus grande précision, de nuit, avec un confort visuel acceptable, tout en augmentant le bien-être en journée : meilleur sommeil (notamment un endormissement ultérieur facilité), moins de fatigue, meilleure cognition et en diminuant les risques de problèmes de santé (ex. : troubles métaboliques, troubles de l'humeur, cancer).

A cet effet, l'invention concerne un dispositif lumineux à LEDs pour la diffusion d'un signal lumineux, sans impact photobiologique sur le rythme circadien ni sur les fonctions cérébrales classiquement perturbées par l'exposition à la lumière pendant la nuit, comme la libération de mélatonine, et tout en permettant un Indice de Rendu des Couleurs suffisant pour la réalisation de tâches nocturnes, ledit dispositif comprenant un logement incorporant au moins une première source lumineuse comportant au moins une LED émettant selon une première longueur d'ondes et une deuxième source lumineuse comportant au moins une LED, ledit logement étant conçu apte à permettre la diffusion du signal émis par lesdites sources lumineuses, ces dernières étant alimentées en énergie au moyen d'un boîtier d'alimentation électrique.

Ledit dispositif est particulier en ce que, d'une part, lesdites sources lumineuses qui constituent ledit dispositif émettent uniquement dans la partie rouge du spectre d'émission et, d'autre part, la première longueur d'ondes émise par ladite première source lumineuse est égale à 670 nm et en ce que ladite deuxième longueur d'ondes émise par ladite deuxième source lumineuse est comprise entre 720 et 750 nm.

On entend ici par « source lumineuse émettant uniquement dans la partie rouge du spectre d'émission », une source émettant à une longueur d'onde supérieure ou égale à 600 nm et à une longueur d'onde inférieure ou égale à 750 nm.

Selon des modes particuliers de réalisation du dispositif lumineux de l'invention :
- ladite deuxième longueur d'ondes émise par ladite deuxième source lumineuse est égale à 740 nm ;
- il comporte une troisième source lumineuse comportant au moins une LED émettant selon une troisième longueur d'ondes, celle-ci étant égale à 660 nm ;
- chacune des sources lumineuses comporte un groupe de LEDs, chacune des LEDs d'un groupe de LEDs émettant selon la même longueur d'ondes ;
- il comporte également des moyens de gestion de la puissance de chacune des LEDs, ou groupes de LEDs, que comportent lesdites sources lumineuses, aptes à permettre un réglage de l'intensité lumineuse émise par chacune desdites sources lumineuses, cette intensité étant comprise entre 25 et 50%, par rapport à une intensité lumineuse maximale égale à 100% ;
- le logement est constitué par un socle surmonté d'une coque à laquelle il est solidarisé ;
- ladite coque est munie d'une poignée intégrée.

La présente invention a également pour objet l'utilisation d'un dispositif lumineux à LEDs, pour la diffusion d'un signal lumineux, sans impact photobiologique sur le rythme circadien ni sur les fonctions cérébrales classiquement perturbées par l'exposition à la lumière pendant la nuit, comme la libération de mélatonine, et tout en permettant un Indice de Rendu des Couleurs suffisant pour la réalisation de tâches nocturnes, ledit dispositif comprenant un logement incorporant au moins une première source lumineuse comportant au moins une LED émettant selon une première longueur d'ondes et une deuxième source lumineuse comportant au moins une LED, ledit logement étant conçu apte à permettre la diffusion du signal émis par lesdites sources lumineuses, ces dernières étant alimentées en énergie au moyen d'un boîtier d'alimentation électrique, ledit dispositif étant constitué de sources lumineuses émettant uniquement dans la partie rouge du spectre d'émission, la première longueur d'ondes émise par ladite première source lumineuse étant égale à 670 nm et ladite deuxième longueur d'ondes émise par ladite deuxième source lumineuse étant comprise entre 720 et 750 nm.

A noter que l'utilisation de ce dispositif lumineux à LEDs selon l'invention ne présente pas d'objectif thérapeutique.

D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux dessins joints en annexe et dans lesquels :
[Fig.1] représente une vue schématisée et en perspective d'une partie d'un mode de réalisation d'un dispositif lumineux à LEDs conforme à l'invention.
[Fig.2] représente une vue schématisée et en perspective d'un mode de réalisation du dispositif lumineux à LEDs conforme à l'invention.

La présente invention concerne un dispositif lumineux 1 à LEDs pour la diffusion d'un signal lumineux.

Le dispositif 1 de l'invention comporte au moins deux sources lumineuses, et, plus préférentiellement, trois sources lumineuses, chacune comprenant une LED, ou un groupe d'une pluralité de LEDs.

Chacune des deux ou trois sources lumineuses, comportant chacune une ou plusieurs LEDs, émet selon une longueur d'ondes spécifique.

De manière particulière au dispositif 1 de la présente invention, lesdites sources lumineuses qui le constituent émettent uniquement dans la partie rouge du spectre d'émission, et chacune desdites sources présente, à cet effet, une longueur d'onde d'émission supérieure ou égale à 600 nm et inférieure ou égale à 750 nm.

En d'autres termes, le dispositif lumineux 1 de l'invention ne présente aucune source lumineuse émettant dans la partie du spectre d'émission émettant une couleur violette, bleue, verte, jaune, ou toute autre couleur du spectre visible autre que le rouge.

Ainsi, tout préférentiellement, le dispositif lumineux 1 de l'invention comporte une première source lumineuse avec une ou plusieurs LEDs émettant selon une longueur d'ondes dite « deep red » de 670 nm.

Ledit dispositif lumineux 1 incorpore, en outre, une deuxième source lumineuse, également avec une ou plusieurs LEDs, émettant, cette fois, selon une longueur d'ondes comprise entre 720 et 750 nm, tout préférentiellement à une longueur d'ondes égale à 740 nm, dite « far red ».

En outre, selon un exemple de réalisation particulièrement avantageux, le dispositif lumineux 1 à LEDs de la présente invention comporte encore une troisième source lumineuse avec au moins une LED, ou avec un groupe de plusieurs LEDs, émettant selon une longueur d'ondes égale à 660 nm.

Un tel dispositif lumineux 1 à LEDs, avec cette combinaison spécifique de longueurs d'ondes, est particulièrement avantageux.

Le dispositif lumineux 1 de l'invention permet, en effet, lors d'une utilisation nocturne, de ne pas influer, de manière néfaste, sur le rythme circadien d'un utilisateur, ni sur les fonctions cérébrales classiquement perturbées par l'exposition à la lumière pendant la nuit, comme la libération de mélatonine, tout en permettant d'obtenir un Indice de Rendu des Couleurs suffisant pour la réalisation de tâches nocturnes qui sont susceptibles de nécessiter une précision d'exécution parfois importante, et amélioré par rapport aux dispositifs connus de l'état de la technique pour lesquels l'IRC est bas.

Ainsi, le dispositif lumineux 1 à LEDs selon la présente invention est particulièrement destiné, sans toutefois que cela soit limitatif, à l'attention de parents de nourrissons ou d'enfants en bas âge, dont les réveils nocturnes sont réguliers, voire fréquents.

Le présent dispositif lumineux 1 permet, alors, à ces utilisateurs, de disposer d'une source lumineuse qui soit, à la fois, sans impact photobiologique sur leur rythme circadien, ni sur les fonctions cérébrales classiquement perturbées par l'exposition à la lumière pendant la nuit, comme la production et la libération de mélatonine par le cerveau, ce qui facilite un endormissement ultérieur et un bien-être en journée, tout en permettant un confort visuel optimal pour la réalisation de tâches nocturnes de précisions, comme, notamment, la lecture de graduations d'une pipette ou d'une notice d'un médicament, indispensables à l'administration de la posologie précise d'un tel produit à un enfant.

L'exemple 1 détaillé ci-dessous présente des résultats de tests effectués permettant d'illustrer l'intérêt et les avantages du présent dispositif 1 de l'invention.

Le dispositif lumineux 1 à LEDs, comportant au moins deux LEDs, ou deux groupes de LEDs, voire trois LEDs ou trois groupes de LEDs, conforme à l'invention, incorpore préférentiellement, au niveau d'un socle 2 que comporte ledit dispositif 1, au moins une carte électronique de circuit imprimé 3 (PCB) destiné à porter lesdites LEDs ou groupes de LEDs.

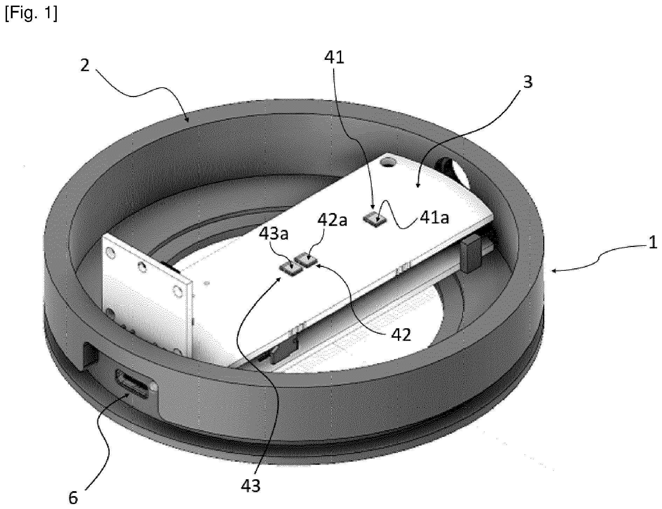

Sur la figure 1 des dessins ci-joints, sur la carte électronique 2 positionnée à l'intérieur du socle 2 que comporte le dispositif 1, sont représentés trois emplacements 41, 42, 43 destinés à l'implantation de trois LEDs 41a, 42a, 43a.

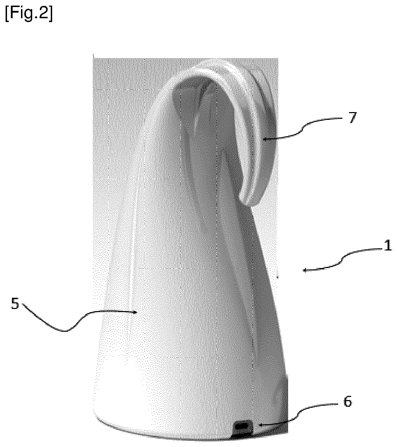

Le dispositif lumineux 1 à LEDs selon l'invention comporte avantageusement un logement, de préférence sous la forme d'une coque 5 visible sur la figure 2, surmontant le socle 2, ladite coque 5 étant avantageusement fabriquée à partir d'un matériau souple, qui peut être translucide ou transparent en sorte de laisser passer le signal émis par les sources lumineuses.

Lorsque le matériau est translucide, celui-ci peut être de couleur blanche, en sorte de laisser passer le signal émis par les sources lumineuses 41a, 42a, 43a, sans modification du spectre du signal.

Ladite coque 5 et ledit socle 2 du dispositif lumineux 1 sont tout préférentiellement solidarisés l'un à l'autre de manière réversible, par des moyens adaptés,

Au sein du logement, avantageusement formé par l'association de ladite coque 5 et du socle 2, sont localisées les sources lumineuses à LEDs, en sorte de permettre une diffusion homogène du signal lumineux qu'elles émettent.

Dans le dispositif lumineux de l'invention, les LEDs ont un cône de diffusion d'au minimum 120°.

Le dispositif lumineux à LEDs 1 selon l'invention comporte également un boîtier d'alimentation conçu pour alimenter en énergie électrique la pluralité de sources lumineuses à LEDs dudit dispositif 1.

Ce boîtier d'alimentation comprend avantageusement une ou plusieurs source(s) d'alimentation électrique, par exemple une batterie ou des piles, cette source, ou ces sources, pouvant préférentiellement être rechargeable(s), par exemple via une prise 6 type micro USB relié à un chargeur destiné à être connecté au secteur.

Le boîtier d'alimentation peut également être conçu apte à relier directement le dispositif lumineux à LEDs 1 de l'invention au secteur au moyen d'une prise de raccordement domestique.

Le boîtier d'alimentation peut également être conçu pour être chargé par induction électromagnétique.

Tout préférentiellement, le dispositif lumineux à LEDs 1 selon l'invention est équipé de moyens de gestion de la puissance des LEDs ou des groupes de LEDs, en sorte de permettre une variation de l'intensité lumineuse émise par celles-ci, entre une intensité lumineuse minimale égale à 12,5% et une intensité lumineuse maximale pouvant aller jusqu'à 100%.

De manière particulièrement avantageuse, dans le dispositif lumineux à LEDs 1 de l'invention, les moyens de gestion de la puissance de chacune des LEDs, ou groupes de LEDs, sont réglés en sorte que les sources lumineuses émettent selon une intensité lumineuse comprise entre 25% et 50%, et de préférence égale à 25 ou égale à 50%, considérant une intensité lumineuse maximale égale à 100%.

La sélection de telles intensités lumineuses sont particulièrement avantageuses, car elles offrent un compromis intéressant permettant à la fois la réalisation de tâches nocturnes parfois minutieuses, tout en évitant l'éblouissement de l'utilisateur ou des utilisateurs.

Tout préférentiellement, et dans un objectif de proposer une ergonomie optimale adapté aux activités de soin du nourrisson ainsi qu'au transport du dispositif lumineux à LEDs 1 selon la présente invention, la coque 5 dudit dispositif 1 est munie une poignée 7, avantageusement intégrée, qui permet de la transporter aisément et de l'accrocher sur différents supports, comme une chaise, ou le lit du nourrisson.

Du fait de sa destination privilégiée, à savoir le soin d'un nourrisson, et de son utilisation principalement nocturne, la matière de la coque du dispositif lumineux 1 de l'invention est tout préférentiellement fabriquée en plastique un matériau plastique souple, apte au contact alimentaire et lavable.

Par ailleurs, un bouton interrupteur, non visible sur les figures, est préférentiellement prévu au niveau de la surface externe de la coque 5 du dispositif 1.

La présente invention a également pour objet l'utilisation du dispositif lumineux à LEDs 1 tel que décrit précédemment, et selon les différents modes de réalisation qui ont été décrits, seuls ou en combinaison, pour la réalisation de tâches nocturnes par un utilisateur, nécessitant un Indice de Rendu des Couleurs suffisants pour leur réalisation, telles que la lecture des graduations d'une pipette ou d'une notice de médicament, sans que l'exposition nocturne audit dispositif n'ait un impact photobiologique sur le rythme circadien dudit utilisateur.

### Exemple 1 : Tests de confort optique du dispositif lumineux à LEDs 1 de l'invention

Les trois types de LEDs utilisées pour la réalisation de ce test ont été choisies sur la base de leur impact neutre sur les rythmes biologiques et notamment sur l'inhibition de la synthèse de mélatonine (une hormone exprimée la nuit) et les paramètres cardiaques (associés à l'éveil et à la vigilance).

Le test a pour objectif la démonstration du fait que les LEDs choisies, utilisées ensemble, permettent un confort visuel adapté aux activités nocturnes, notamment de nourrissage ou de soins des parents d'enfants en bas âges ou de nourrissons.

Les LEDs qui ont été utilisées sont les suivantes, avec précision concernant la longueur d'ondes de la lumière émise :
- GH CSSRM5.24-V8V9-1-1-700-R33 : 660 nm
- L128-DRD1003500000 : 670 nm
- L128-FRD1003500000 : 720-750 nm

Les circuits imprimés (PCB) à LEDs suivant ont été testés : 4 PCB ont été testés, chacun avec la combinaison des trois LEDs susmentionnées, à quatre intensités différentes (échelle interne) : 100% - 50% - 25% -12,5%.

Le diffuseur de lumière utilisé correspond à un prototype du déposant imprimé en 3D en un matériau élastomère thermoplastique (TPE) de couleur blanche et translucide, ledit matériau étant apte au contact alimentaire.

Le test a été réalisé par quatre personnes, dans les conditions décrites ci-après.

L'étude a été menée pendant la nuit (entre 3 et 5h du matin). En effet, l'état physiologique du système de transmission rétinien est lui-même sous l'effet de l'horloge circadienne et les connexions entre les différents systèmes de photorécepteurs ne sont pas les mêmes le jour et la nuit.

Par ailleurs, les tests ont été réalisés après une période d'adaptation à l'obscurité de plus de 30 min permettant de reproduire les conditions d'un réveil nocturne habituel.

Il était donc important de faire les tests sur des yeux adaptés à l'obscurité.

Enfin, les parents de nourrissons ou d'enfants en bas âge seront sans doute réveillés au milieu de leur sommeil, ce qui peut avoir des conséquences sur leur état de vigilance ce qui a également dû être pris en compte pour la réalisation des tests.

### Paramètres observés

- Distinction de l'environnement direct en statique ;
- Déplacement à une allure modérée ;
- Lecture des graduations d'une pipette de médicament tenue à proximité de la lampe ;
- Lecture de la notice d'utilisation d'un médicament tenue à proximité de la lampe ;
- Distinction du téton - peau blanche (important pour mise au sein lors de l'allaitement).

### Résultats

| | **12,5%** | **25%** | **50%** | **100%** |
|---|---|---|---|---|
| **Distinction de l'environnement direct en statique** | 2-3 m | 4 m | 4-5 m | Ok |
| **Déplacement à une allure modérée** | Non | Ok | Ok | Ok |
| **Lecture des graduations d'une pipette** | Non | Ok | Ok | Ok |
| **Lecture de la notice d'utilisation d'un médicament** | Non | Ok | Ok | Ok |
| **Distinction du téton** | Ok | Ok | Ok | Ok |
| **Eblouissant** | non | non | oui-non | oui |

### Conclusions :

Dans des conditions proches de celles rencontrées par les nouveaux parents en plein réveil, le confort visuel apporté par la combinaison de LEDs du dispositif lumineux 1 de l'invention permet d'effectuer les tests correspondant aux activités de maternage nocturne : distinction de l'environnement proche, déplacement à l'intérieur du domicile, lecture de graduations (biberon, médicaments), distinction du téton.

Ces tests ont par ailleurs permis de mettre en évidence que, dans certains cas, il était préférable de choisir une puissance de fonctionnement des sources lumineuses permettant d'obtenir une intensité lumineuse comprise entre 25 et 50%.

En effet, une intensité lumineuse égale à 12,5% ne permet pas d'effectuer certaines tâches, tandis que l'intensité maximale de 100%, peut avoir un effet éblouissant.

## Revendications

1. Dispositif lumineux à LEDs (1) pour la diffusion d'un signal lumineux, sans impact photobiologique sur le rythme circadien ni sur les fonctions cérébrales classiquement perturbées par l'exposition à la lumière pendant la nuit, comme la libération de mélatonine, tout en permettant un Indice de Rendu des Couleurs suffisant pour la réalisation de tâches nocturnes, ledit dispositif (1) comprenant un logement incorporant au moins une première source lumineuse comportant au moins une LED émettant selon une première longueur d'ondes et une deuxième source lumineuse comportant au moins une LED, ledit logement étant conçu apte à permettre la diffusion du signal émis par lesdites sources lumineuses, ces dernières étant alimentées en énergie au moyen d'un boîtier d'alimentation électrique, ledit dispositif (1) étant **caractérisé en ce que** lesdites sources lumineuses émettent uniquement dans la partie rouge du spectre d'émission, la première longueur d'ondes émise par ladite première source lumineuse est égale à 670 nm et **en ce que** ladite deuxième longueur d'ondes émise par ladite deuxième source lumineuse est comprise entre 720 et 750 nm.

2. Dispositif lumineux à LEDs (1) pour la diffusion d'un signal lumineux selon la revendication 1 **caractérisé en ce que** ladite deuxième longueur d'ondes émise par ladite deuxième source lumineuse est égale à 740 nm.

3. Dispositif lumineux à LEDs (1) pour la diffusion d'un signal lumineux selon la revendication 1 ou la revendication 2 **caractérisé en ce qu'**il comporte une troisième source lumineuse comportant au moins une LED émettant selon une troisième longueur d'ondes, celle-ci étant égale à 660 nm.

4. Dispositif lumineux à LEDs (1) pour la diffusion d'un signal lumineux selon l'une quelconque des revendications précédentes **caractérisé en ce que** chacune des sources lumineuses comporte un groupe de LEDs, chacune des LEDs d'un groupe de LEDs émettant selon la même longueur d'ondes.

5. Dispositif lumineux à LEDs (1) pour la diffusion d'un signal lumineux selon l'une quelconque des revendications précédentes **caractérisé en ce qu'**il comporte également des moyens de gestion de la puissance de chacune des LEDs, ou groupes de LEDs, que comportent lesdites sources lumineuses, aptes à permettre un réglage de l'intensité lumineuse émise par chacune desdites sources lumineuses, cette intensité étant comprise entre 25 et 50%, par rapport à une intensité lumineuse maximale égale à 100%.

6. Dispositif lumineux à LEDs (1) pour la diffusion d'un signal lumineux selon l'une quelconque des revendications précédentes **caractérisé en ce que** le logement est constitué par un socle (2) surmonté d'une coque (5) à laquelle il est solidarisé.

7. Dispositif lumineux à LEDs (1) pour la diffusion d'un signal lumineux selon la revendication précédente **caractérisé en ce que** ladite coque (5) est munie d'une poignée intégrée (7).

8. Utilisation du dispositif lumineux à LEDs (1) selon l'une quelconque des revendications précédentes pour la réalisation de tâches nocturnes par un utilisateur, nécessitant un Indice de Rendu des Couleurs suffisants, telles que la lecture des graduations d'une pipette ou d'une notice de médicament, sans impact photobiologique sur le rythme circadien dudit utilisateur ni sur les fonctions cérébrales classiquement perturbées par l'exposition à la lumière pendant la nuit, comme la libération de mélatonine. 1
